# FASCICULE DE BREVET EUROPEEN

(11) **EP 1 003 901 B1**
(45) Date de publication et mention de la délivrance du brevet: **12.11.2003**
(21) Numéro de dépôt: 98936484.9
(22) Date de dépôt: 08.07.1998
(51) Int. Cl.: C12P 19/44, C12P 19/02, C12P 7/64

(54) **PROCEDE DE SYNTHESE ENZYMATIQUE DE SUCROESTERS**
VERFAHREN ZUR ENZYMATISCHEN SYNTHESE VON SACCHARID-ESTERN
METHOD FOR ENZYMATIC SYNTHESIS OF SUCROSE ESTERS

(30) Priorité: 09.07.1997 FR 9708916
(43) Date de publication de la demande: 31.05.2000
(73) Titulaire: Cognis France S.A., 31360 Saint Martory (FR)
(72) Inventeur: PAULY, Gilles, F-54000 Nancy (FR)
(74) Mandataire: Nuss, Pierre
(86) Numéro de dépôt international: FR9801479
(87) Numéro de publication internationale: WO99002722

(56) Documents cités:
- EP-A- 0 087 404
- EP-A- 0 413 307
- EP-A- 0 507 323
- DATABASE WPI Section Ch, Week 9737 Derwent Publications Ltd., London, GB; Class D16, AN 97-397039 XP002060488 & JP 09 173091 A (LION CORP) , 8 juillet 1997
- DATABASE WPI Section Ch, Week 9734 Derwent Publications Ltd., London, GB; Class D16, AN 97-367073 XP002060489 & JP 09 154595 A (LION CORP), 17 juin 1997
- DATABASE WPI Section Ch, Week 9402 Derwent Publications Ltd., London, GB; Class D16, AN 94-011028 XP002060490 & JP 05 317064 A (KAO CORP) , 3 décembre 1993

## Description

La présente invention concerne le domaine des procédés de synthèse de substances et a pour objet un procédé de synthèse enzymatique de sucroesters.

Les sucroesters sont des tensioactifs résultant de la combinaison par liaison ester d'un sucre et d'un acide gras. En variant la nature du sucre et la longueur de la chaîne grasse, il est possible d'obtenir un ensemble de molécules possédant une gamme très large de balances hydrophile-lipophile et donc de propriétés fonctionnelles. On peut ainsi produire des tensioactifs moussants, peu-moussants, fluidifiants, solubilisants ou émulsionnants.

Ces produits constituent des ingrédients naturels intéressant les secteurs de la détergence, de la cosmétique, de la pharmacie et de l'agroalimentaire.

Les sucroesters commercialisés actuellement sont généralement produits par synthèse chimique.

Ce sont généralement des mélanges complexes de différents composés, résultant de la non spécificité des réactions chimiques de condensation entre les sucres et les acides gras. Les températures et pressions élevées nécessaires pour ces réactions conduisent donc également à des réactions parasites et des phénomènes de coloration des produits.

Les sucroesters peuvent aussi être obtenus par des réactions de condensation catalysées par des enzymes, par exemple des lipases. Ces réactions enzymatiques ont l'avantage d'être plus spécifiques que les synthèses chimiques et de se dérouler à des températures et des pressions ordinaires.

SEINO et al ont proposé (J. Am Oil Chem. Soc. 61, 1761-1765, 1984) d'estérifier des sucres (sucrose, glucose, fructose, sorbitol) avec des acides gras (acide stéarique, oléique, linoléïque) en présence d'une enzyme dans un milieu aqueux. Mais cette technologie ne permet pas d'obtenir de quantités significatives de sucroesters.

En outre, les documents WO 90/09451 et WO 94/01575 décrivent des procédés d'obtention de sucroesters en absence totale d'eau et sans utilisation de solvant. Cette technologie nécessite cependant une alkylation préalable des sucres pour permettre leur solubilisation dans la phase grasse. Cette technologie de production d'esters d'alkylsucres et d'acides gras présente aussi l'inconvénient de conduire à une préparation contenant un excès de matière grasse non transformée et donc de nécessiter des opérations complexes de purification si on veut obtenir des niveaux de pureté élevés pour les sucroesters résultants. Par ailleurs, la viscosité élevée des milieux réactionnels utilisés complique la mise en oeuvre des opérations.

ZAKS A. et KLIBANOV A.M. (Proc. Natl. Acad. Sci. USA, 82, 3192-3196, 1985) ont décrit, en premier, la synthèse enzymatique de sucroesters d'acides gras dans un solvant organique. Cependant, la pyridine utilisée est un solvant toxique prohibé pour des applications industrielles.

De plus, l'utilisation de solvants organiques de type alcools tertiaires pour réaliser les synthèses enzymatiques de sucroesters a également déjà été proposée.

Ainsi, le document FR-A-2 646 439 et KHALED et al (Biotech. Letters, 13, 167-172, 1991) décrivent les synthèses d'oléate de fructose, de sorbose, de sorbitol et de mannitol. ainsi que de palmitate de fructose en utilisant une lipase immobilisée et le méthyl-2 butanol-2 comme solvant. Toutefois, les performances de ces procédés de synthèse demeurent peu élevées : des concentrations finales de sucroesters de l'ordre de 20 g/l, des rendements de conversion des sucres ne dépassant pas 50 % et des productivités spécifiques inférieures à 0,01 g de sucroesters produits par heure et par gramme de catalyseur. En outre, les excès d'acides gras utilisés nécessitent des opérations complexes de fractionnement des mélanges de sucroesters et de corps gras obtenus.

Par ailleurs, DUCRET et al (Biotechnol. Bioeng. 48, 214-221, 1995) décrivent la synthèse, par une lipase immobilisée d'oléate de fructose et de glucose dans du méthyl-2 butanol-2, en maintenant un vide partiel au dessus du milieu réactionnel. Cette technologie conduit à des degrés de conversion plus élevés de 93 % pour l'oléate de fructose et de 70 % pour l'oléate de glucose. Cependant, les concentrations finales de sucroesters demeurent peu élevées (23 g/l pour l'oléate de fructose, 17g/l pour l'oléate de glucose), et les productivités spécifiques sont inférieures à 0,1 g de sucroester par heure et par gramme de catalyseur. En outre, cette technologie ne donne pas de synthèse d'esters de saccharose et de lactose.

Enfin, WOUDENBERG et al (Biotechnol. Bioeng. 49, 328-333, 1996) décrivent des estérifications enzymatiques de disaccharides, comme le sucrose, avec de l'éthylbutanoate et de l'éthyldodécanoate en utilisant une lipase immobilisée et le méthyl-2 butanol-2 comme solvant. Mais les concentrations finales et les productivités (0,04 g/h.g) spécifiques obtenues sont très faibles.

La présente invention a notamment pour but de pallier l'ensemble des inconvénients précités et de proposer un procédé de synthèse enzymatique de sucroesters qui, comparé aux procédés connus précédemment mentionnés, permet d'atteindre des performances nettement supérieures en termes de concentrations finales de sucroesters, de rendements de conversions (à la fois pour les sucres et pour les corps gras présents à l'origine) et de productivités spécifiques, tout en réduisant les opérations complexes et fastidieuses de purification après synthèse.

A cet effet, la présente invention a pour objet un procédé de synthèse enzymatique de sucroesters, caractérisé en ce qu'il consiste à introduire, dans un réacteur adapté et de manière à former un milieu réactionnel, des quantités déterminées d'un solvant organique, d'un sucre ou d'un dérivé de sucre, d'un composé donneur d'acyles et d'un catalyseur enzymatique, la quantité d'au moins un des constituants dudit milieu réactionnel étant déficitaire, à ajouter de manière contrôlée au cours de la réaction des quantités supplémentaires du ou des constituant(s) déficitaire(s) et, enfin, à purifier les sucroesters produits au moins par séparation des particules enzymatiques (par exemple par décantation, filtration ou centrifugation) et du solvant (par exemple par évaporation, distillation ou filtration membranaire).

Le procédé selon l'invention consiste plus précisément, durant le déroulement de la réaction de synthèse, à ajouter au milieu réactionnel, de façon continue ou intermittente, des quantités additionnelles déterminées de sucre ou de dérivé de sucre, sous forme solide ou sous forme de solution liquide, de solvant, de catalyseur enzymatique sous forme soluble ou immobilisée et/ou de composé donneur d'acyles, seul ou solubilisé dans le solvant.

Ainsi, la différence principale entre le procédé selon l'invention et les procédés connus précédemment décrits réside dans la conduite de la réaction enzymatique.

En effet, dans les procédés de synthèse enzymatique de sucroesters connus, la totalité des quantités des constituants du milieu réactionnel est introduite dans le réacteur en début de réaction.

Or, selon l'invention, seule une quantité initiale déterminée d'un ou de plusieurs des constituants du milieu réactionnel est initialement introduite dans le récipient de réaction et des quantités supplémentaires du ou des constituants partiellement introduits à l'origine sont ajoutées au cours du déroulement de la réaction de synthèse.

En contrôlant la quantité des réactifs additionnés, il est possible de commander l'évolution au cours du temps de la composition du milieu réactionnel et, par ce biais, d'orienter la réaction enzymatique vers une production maximale de monoesters et/ou de diesters, en limitant les réactions parasites.

Conformément à l'invention, la conduite de la réaction est menée de manière à limiter en premier lieu les phénomènes d'inhibition de la réaction enzymatique que l'on observe en présence de fortes concentrations de sucres ou de donneurs d'acyles.

Ainsi, il a été constaté que, en vue d'obtenir une concentration finale élevée en sucroesters, il est préférable de ne pas introduire initialement dans le réacteur l'ensemble des quantités de réactifs nécessaires, mais au contraire de les apporter progressivement, de manière contrôlée, au cours du déroulement de la réaction en évitant d'atteindre des niveaux de concentration inhibitrice de la réaction enzymatique.

La conduite de la réaction est menée de manière à contrôler tout au long de son déroulement le rapport molaire [sucre(s) ou dérivé(s) de sucre(s)/ donneur d'acyle(s)].

De manière avantageuse, ce rapport molaire peut être compris entre 0,01 et 10,00, préférentiellement entre 0,02 et 2,00, en fonction des composants formant le couple sucre(s) ou dérivé(s) de sucre(s)/ donneur d'acyle(s) concerné.

En fixant dans le milieu réactionnel des valeurs de rapport molaire contenues dans les plages précitées. il est possible d'obtenir soit des vitesses de réaction plus élevées, soit des proportions maximales de monoesters ou de diesters de sucres.

Ce rapport molaire pourra, en contrôlant les natures et les quantités de réactifs ajoutées au cours du temps, soit être maintenu à une valeur constante au cours de toute la durée de la réaction, soit être soumis à une variation contrôlée de manière à suivre un profil de variation déterminé au cours du temps, tout en étant compris dans la fourchette de valeurs précitée tout au long de la réaction.

En vue d'optimiser le déroulement de la réaction de synthèse, il est possible de procéder éventuellement à un soutirage intermittent ou continu d'au moins un des constituants du milieu réactionnel, le ou lesdits constituant(s) soutiré(s) pouvant éventuellement être réintroduit(s) dans le réacteur après fractionnement.

Selon un mode de réalisation de l'invention, il peut être prévu de réaliser un soutirage intermittent ou continu du milieu réactionnel complet, un ou plusieurs constituant(s) dudit milieu soutiré pouvant être réinjecté(s) dans le réacteur après fractionnement.

Le récipient de réaction ou réacteur utilisé pour la mise en oeuvre du procédé selon l'invention est avantageusement équipé d'une régulation de température, d'une régulation de pression, de moyens d'ajout de réactifs et de moyens de soutirage de produits.

Au cours du déroulement de la réaction de synthèse, la température est avantageusement régulée entre 20° C et 100° C, la pression partielle au-dessus du milieu réactionnel est avantageusement régulée entre 10 mbars (10³ Pa) et 1000 mbars (10⁵ Pa) et ledit milieu réactionnel est avantageusement soumis à une agitation douce.

En vue d'obtenir des préparations résultant de sucroesters présentant des puretés élevées, il peut en outre être prévu de mettre en oeuvre des opérations finales supplémentaires de fractionnement, par exemple, en éliminant les sucres ou les corps gras résiduels par extraction par des solvants organiques ou par des fluides supercritiques ou en fractionnant les monoesters et/ou diesters produits par précipitations(s) ou séparation(s) chromatographique(s).

Le sucre ou dérivé de sucre utilisé dans le cadre de la présente invention peut consister en un composé quelconque répondant aux types précités, en particulier les composés de la famille des oses.

Selon un mode de réalisation préférentiel de l'invention, le sucre ou le dérivé de sucre est choisi dans le groupe formé par le fructose, le glucose, le saccharose, le tréhalose, les dérivés éthylés et méthylés de ces sucres et les composés structurellement analogues, tels que les polyols.

Le composé donneur d'acyle(s) est choisi parmi les acides gras connus et peut être préférentiellement sélectionné dans le groupe formé par les acides gras, saturés ou insaturés, à nombre pair ou impair d'atomes de carbones, linéaires ou branchés et dont le nombre d'atomes de carbones est supérieur à 4, les esters d'acides gras, les monoglycérides, diglycérides, triglycérides et les huiles.

En tant que solvant organique, il est possible d'utiliser tout composé organique ou tout mélange de composés organiques permettant une solubilisation totale ou partielle des sucres ou dérivés de sucres et des donneurs d'acyle(s) choisis.

Ainsi, le ou les solvant(s) pourront être notamment sélectionnés parmi les substances suivantes : méthanol, éthanol, propanol, butanol, acétone, propanone, butanone, pentanone 2, 1,2 éthanediol, 2,3 butanediol, dioxane, acétonitrile, méthyl 2 butanol 2, tertiobutanol, méthyl-2-propanol et hydroxy-4-méthyl-2-pentanone, ou un mélange de deux ou de plusieurs de ces solvants.

Le catalyseur enzymatique mis en oeuvre doit bien entendu induire et favoriser le transfert d'un groupement acyle d'un donneur d'acyle(s) à un sucre ou un dérivé de sucre et peut consister avantageusement en une protéase ou une lipase, préférentiellement immobilisée sur un support.

Compte tenu des différentes caractéristiques mentionnées ci-dessus, plusieurs variantes de réalisation de l'invention peuvent être envisagées, fonction notamment de la nature des réactifs utilisés et des buts privilégiés à atteindre.

Ainsi, il est possible, selon une première variante de réalisation, d'envisager un procédé de synthèse avec ajout de donneur d'acyle et de solvant au cours de la réaction.

Dans ce cas, le réacteur contient initialement le solvant, la quantité totale de sucre ou de dérivé de sucre (généralement entre 10 g/l et 200 g/l) nécessaire pour obtenir la quantité finale de sucroester souhaitée, la quantité de donneur d'acyle correspondant au rapport molaire (sucre dissout/donneur d'acyle) initial recherché (généralement entre 1 g/l et 500 g/l) et l'enzyme apportée sous forme soluble ou immobilisée (de 1 g/l à 100 g/l, préférentiellement entre 5 g/l et 20 g/l).

Au cours du déroulement de la réaction, on ajoute, d'une part, du solvant de manière à compenser les pertes par évaporation et à maintenir une quantité de solvant relativement constante et, d'autre part, du donneur d'acyle en quantité par unité de temps adaptée pour maintenir le rapport molaire (sucre dissout/donneur d'acyle) à la valeur recherchée.

Ainsi, lorsqu'il est avantageux de maintenir ce rapport molaire constant tout au long de la réaction, le donneur d'acyle est ajouté à une vitesse égale à sa vitesse de consommation par la réaction, cette vitesse de consommation pouvant être déterminée par une étude cinétique préalable de la réaction enzymatique mise en oeuvre. La quantité par unité de temps de donneur d'acyle à ajouter au cours de la réaction est généralement comprise entre 0,01 et 10 grammes de donneur d'acyle par heure et par gramme de catalyseur enzymatique présent dans le réacteur.

Conformément à une seconde variante de réalisation de l'invention, le procédé de synthèse peut également être effectué avec ajout de sucre et de solvant.

Dans ce second cas, le réacteur contient initialement le solvant, la quantité totale de donneur d'acyle (généralement entre 1 g/l et 500 g/l) nécessaire pour obtenir la quantité finale de sucroester souhaitée, la quantité de sucre ou de dérivé de sucre correspondant au rapport molaire (sucre dissout/donneur d'acyle) initial recherché (généralement entre 1 g/l et 200 g/l) et l'enzyme apportée sous forme soluble ou immobilisée (de 1 g/l à 100 g/l, préférentiellement entre 5 g/l et 20 g/l).

Au cours du déroulement de la réaction, on ajoute, d'une part, du solvant de manière à compenser les pertes par évaporation et à maintenir une quantité de solvant relativement constante et, d'autre part, du sucre ou du dérivé de sucre en quantité par unité de temps adaptée pour maintenir le rapport molaire (sucre dissout/donneur d'acyle) à la valeur recherchée.

Ainsi, lorsqu'il est avantageux de maintenir cc rapport molaire constant tout au long de la réaction, le sucre ou dérivé de sucre est ajouté à une vitesse égale à sa vitesse de consommation par la réaction, cette vitesse de consommation pouvant être déterminée par une étude cinétique préalable de la réaction enzymatique mise en oeuvre. La quantité par unité de temps de sucre ou de dérivé de sucre à ajouter au cours de la réaction est généralement comprise entre 0,01 et 10 grammes de sucre ou de dérivé de sucre par heure et par gramme de catalyseur enzymatique présent dans le réacteur.

Selon une troisième variante de réalisation de l'invention, le procédé de synthèse peut également être effectué en ajoutant du sucre (ou du dérivé de sucre), du donneur d'acyle et du solvant.

Dans ce troisième cas, le réacteur contient initialement le solvant, le sucre à une concentration variable (de préférence supérieure à la solubilité du sucre dans le solvant) et la quantité de donneur d'acyle correspondant au rapport molaire (sucre dissout/donneur d'acyle) initial recherché, ainsi que l'enzyme apportée sous forme soluble ou immobilisée.

Au cours du déroulement de la réaction, on procède, d'une part, à l'ajout de solvant en vue de compenser les pertes par évaporation et, d'autre part, à l'ajout de sucre (ou de dérivé de sucre) et de donneur d'acyle en quantités par unités de temps déterminées de manière à maintenir le rapport molaire de ces deux constituants à la valeur recherchée.

Lorsqu'il est avantageux de maintenir ce rapport molaire constant tout au long de la réaction, le sucre (ou dérivé de sucre) et le donneur d'acyle sont ajoutés en des quantités par unités de temps respectivement égales à leurs vitesses de consommation par la réaction, ces vitesse de consommation pouvant être déterminées par une étude cinétique préalable de la réaction enzymatique mise en oeuvre.

Conformément à une quatrième variante de réalisation de l'invention, le procédé de synthèse continu peut également être conduit avec ajout et soutirage de sucre (ou de dérivé de sucre), de donneur d'acyle, et/ou de solvant, ainsi qu'éventuellement de catalyseur enzymatique.

Dans ce quatrième cas, le réacteur contient initialement le solvant, le sucre à une concentration variable (de préférence supérieure à la solubilité du sucre dans le solvant) et la quantité de donneur d'acyle correspondant au rapport molaire (sucre dissout/donneur d'acyle) initial recherché, ainsi que l'enzyme apportée sous forme soluble ou immobilisée.

Au cours du déroulement de la réaction, on soutire de façon continue ou intermittente du milieu réactionnel, l'enzyme pouvant être retenue à l'intérieur du réacteur lorsqu'il est sous forme immobilisée.

Après séparation, le solvant, et éventuellement le sucre et/ou le donneur d'acyle, peuvent être recyclés dans le réacteur.

Tout au long du déroulement de la réaction, on procède, d'une part, à l'ajout de solvant en vue de compenser les pertes par évaporation et par soutirage et, d'autre part, à l'ajout de sucre (ou de dérivé de sucre) et de donneur d'acyle en quantités par unités de temps déterminées de manière à maintenir le rapport molaire de ces deux constituants à la valeur recherchée.

Lorsqu'il est avantageux de maintenir ce rapport molaire constant tout au long de la réaction, le sucre (ou dérivé de sucre) et le donneur d'acyle sont ajoutés en des quantités par unités de temps respectivement égales à leurs vitesses de consommation par la réaction et de soutirage.

A titre d'exemples non limitatifs, on décrira ci-après différents modes de réalisation pratiques de l'invention.

### Exemple 1 : Production d'oléate de fructose avec ajout intermittent de fructose

On introduit initialement dans un réacteur en verre 1 litre de méthyl-2 butanol-2, 25 grammes de fructose, 106 grammes d'oléate de méthyle et 5 grammes de particules de lipase immobilisées du type connu sous la désignation commerciale Novozym. La température du réacteur est régulée à 60° C, la pression à 200 mbars (2 x 10⁴ Pa), et l'agitation à 200 rpm (rotations par minute). Après 8 heures de réaction, on ajoute 25 grammes de fructose, et après 16 heures de réaction, on ajoute à nouveau 25 grammes de fructose. Le rapport molaire fructose/oléate de méthyle, initialement fixé à une valeur de 0,38, est maintenu au cours de la réaction entre 0,12 et 5,00.

Au cours du déroulement de la réaction de synthèse, on obtient les productions suivantes d'oléate de fructose :
- après 8 heures de réaction, on relève 45 g/1 de monoester et 5 g/l de diester. Ceci correspond à une conversion globale de 78 % du fructose et une productivité spécifique de 1,2 g de sucroester par heure et par gramme de particules enzymatiques.
- après 16 heures de réaction, on relève 65 g/1 de monoester et 10 g/1 de diester. Ceci correspond à une conversion globale de 58 % du fructose et une productivité spécifique de 0,9 g de sucroester par heure et par gramme de particules enzymatiques.
- après 20 heures de réaction, on relève 80 g/l de monoester et 10 g/l de diester. Ceci correspond à une conversion globale de 46 % du fructose et une productivité spécifique de 0,9 g de sucroester par heure et par gramme de particules enzymatiques.

### Exemple 2 : Production d'oléate de fructose avec ajout continu de fructose et d'oléate de méthyle

On introduit initialement dans un réacteur en verre 500 ml de méthyl-2 butanol-2, 5 grammes de fructose, 8,3 grammes d'oléate de méthyle et 10 grammes de particules de lipase immobilisées du type connu sous la désignation commerciale Novozym. La température du réacteur est régulée à 60° C, la pression à 200 mbars (2 x 10⁴ Pa), et l'agitation à 200 rpm (rotations par minute). Au cours de la réaction, on ajoute avec un débit de 1 ml/mn une solution de méthyl-2 butanol-2 renfermant 55 mM de fructose et 55 mM d'oléate de méthyle. Le rapport molaire fructose/oléate de méthyle, initialement fixé à une valeur de 1,0, est maintenu au cours de la réaction entre 0,5 et 1,5.

Après 20 heures de réaction, on obtient 40 grammes de monoester d'oléate de fructose, sans quantité détectable de diester. Ceci correspond à une conversion globale de 95 % du fructose et de 95 % de l'oléate de méthyle, et une productivité spécifique de 0,15 gramme de sucroester par heure et par gramme de particules enzymatiques.

### Exemple 3 : Production d'oléate de fructose avec ajout continu d'oléate de méthyle

On introduit initialement dans un réacteur en verre 500 ml de méthyl-2 butanol-2, 12,5 grammes de fructose, 8,3 grammes d'oléate de méthyle et 10 grammes de particules de lipase immobilisées du type connu sous la désignation commerciale Novozym. La température du réacteur est régulée à 60° C, la pression à 200 mbars (2 x 10⁴ Pa), et l'agitation à 200 rpm (rotations par minute). Au cours de la réaction, on ajoute avec un débit de 0.66 ml/mn une solution de méthyl-2 butanol-2 renfermant 206 mM d'oléate de méthyle. Le rapport molaire fructose/oléate de méthyle, initialement fixé à une valeur de 2,0, est maintenu au cours de la réaction entre 1,0 et 2,0.

Après 18 heures de réaction, on obtient 60 grammes de monoester d'oléate de fructose, sans quantité détectable de diester. Ceci correspond à une conversion globale de 95 % du fructose et de 95 % de l'oléate de méthyle, et une productivité spécifique de 0,3 gramme de sucroester par heure et par gramme de particules enzymatiques.

### Exemple 4 : Production de pélargonate de tréhalose avec un ajout continu d'acide pélargonique

On introduit initialement dans un réacteur en verre 465 ml de méthyl-2 butanol-2, 14 grammes de tréhalose, 72 mmole d'acide pélargonique et 10 grammes de particules de lipase immobilisées du type connu sous la désignation commerciale Novozym. La température du réacteur est régulée à 60° C, la pression à 200 mbars (2 x 10⁴ Pa), et l'agitation à 200 rpm (rotations par minute). Au cours de la réaction et pendant 12 heures consécutives, on ajoute avec un débit de 0,66 ml/mn, un mélange de 32 ml de méthyl-2 butanol-2 et de 468 ml d'acide pélargonique. Le rapport molaire fructose/acide pélargonique, initialement fixé à une valeur de 0,09, est maintenu au cours de la réaction entre 0,01 et 0,09.

Après 18 heures de réaction, on obtient 3 grammes de monoester de pélargonate de tréhalose et 22 g/l de diester. Ceci correspond à une conversion globale de 53 % du tréhalose et de 3 % de l'acide pélargonique, et une productivité spécifique de 0,13 gramme de sucroester par heure et par gramme de particules enzymatiques.

Après 48 heures de réaction, on obtient 2 grammes de monoester de pélargonate de tréhalose et 25 g/l de diester. Ceci correspond à une conversion globale supérieure à 98 % du tréhalose et de 3 % de l'acide pélargonique, et une productivité spécifique de 0,06 gramme de sucroester par heure et par gramme de particules enzymatiques.

Comme le montrent à titre illustratif les différents exemples décrits ci-dessus, le procédé de synthèse enzymatique de sucroesters selon l'invention présente de nombreux avantages par rapport aux procédés similaires connus et permet notamment d'atteindre les performances suivantes, résultant en particulier en des avantages économiques notables:
- des concentrations finales de sucroesters pouvant atteindre 90 g/l ;
- des rendements de conversion supérieurs à 95 %, à la fois pour le sucre ou dérivés de sucres et le composé donneur d'acyle(s);
- des sélectivités de réaction soit de plus de 99 % de monoesters, soit de plus de 90 % de diesters ;
- des productivités spécifiques allant jusqu'à 0,7 g de sucroesters par heure et par gramme de particules enzymatiques.

En plus des performances précitées, il convient également de noter que, grâce à l'invention, on obtient une composition bien définie en sucroesters et donc une qualité de produits supérieure.

En outre, l'absence de réactions parasites et de phénomènes de coloration aux températures utilisées (généralement autour de 60° C) permet de minimiser les opérations de purification et la génération d'effluents, améliorant d'avantage encore la compétitivité économique du procédé selon l'invention.

Enfin, ce dernier se distingue également des procédés connus par sa très grande polyvalence, qui autorise son application à une gamme très large de sucres (monosaccharides et disaccharides) et donneurs d'acyles (acides gras, esters d'acides gras, huiles, esters d'huiles) et, par voie de conséquence, la génération d'une gamme très large de nouveaux sucroesters.

Bien entendu, l'invention n'est pas limitée au mode de réalisation décrit. Des modifications restent possibles, notamment du point de vue de la constitution des divers éléments ou par substitution d'équivalents techniques, sans sortir pour autant du domaine de protection de l'invention.

## Revendications

1. Procédé de synthèse enzymatique de sucroesters, **caractérisé en ce qu'**il consiste à introduire, dans un réacteur adapté et de manière à former un milieu réactionnel, des quantités déterminées d'un solvant organique, d'un sucre ou d'un dérivé de sucre, d'un composé donneur d'acyles et d'un catalyseur enzymatique, la quantité d'au moins un des constituants dudit milieu réactionnel étant déficitaire, à ajouter de manière contrôlée au cours de la réaction des quantités supplémentaires du ou des constituant(s) déficitaire(s) et, enfin, à purifier les sucroesters produits au moins par séparation des particules enzymatiques et du solvant.

2. Procédé selon la revendication 1, **caractérisé en ce qu'**il consiste à ajouter au milieu réactionnel, de façon continue ou intermittente, des quantités additionnelles déterminées de sucre ou de dérivé de sucre, sous forme solide ou sous forme de solution liquide, de solvant, de catalyseur enzymatique sous forme soluble ou immobilisée et/ou de composé donneur d'acyles, seul ou solubilisé dans le solvant.

3. Procédé selon l'une quelconque des revendications 1 et 2, **caractérisé en ce qu'**il consiste à procéder à un soutirage intermittent ou continu d'au moins un des constituants du milieu réactionnel, le ou lesdits constituant(s) soutiré(s) étant réintroduit(s) dans le réacteur après fractionnement.

4. Procédé selon la revendication 3, **caractérisé en ce qu'**il consiste à réaliser un soutirage intermittent ou continu du milieu complet, un ou plusieurs constituant(s) dudit milieu soutiré étant réinjecté(s) dans le réacteur après fractionnement.

5. Procédé selon l'une quelconque des revendications 1 à 4, **caractérisé en ce qu'**il consiste à contrôler le rapport molaire [sucre(s) ou dérivé(s) de sucre(s)/donneur d'acyle(s)] dans le milieu réactionnel, de telle manière que ce rapport molaire soit compris entre 0,01 et 10,00, préférentiellement entre 0,02 et 1,00, tout au long du déroulement dudit procédé, ce en fonction des composants du couple sucre(s) ou dérivé(s) de sucre(s)/donneur d'acyle(s) concerné.

6. Procédé selon l'une quelconque des revendications 1 à 5, **caractérisé en ce que**, au cours de la réaction, la température est régulée entre 20° C et 100° C, la pression partielle au-dessus du milieu réactionnel est régulée entre 10 mbars et 1000 mbars et ledit milieu réactionnel est soumis à une agitation douce.

7. Procédé selon l'une quelconque des revendications 1 à 6, **caractérisé en ce qu'**il consiste à éliminer les sucres ou les corps gras résiduels par extraction par des solvants organiques ou par des fluides supercritiques.

8. Procédé selon l'une quelconque des revendications 1 à 7, **caractérisé en ce qu'**il consiste à fractionner les monoesters et/ou diesters produits par précipitations(s) ou séparation(s) chromatographique(s).

9. Procédé selon l'une quelconque des revendications 1 à 8, **caractérisé en ce que** le sucre ou le dérivé de sucre est choisi dans le groupe formé par le fructose, le glucose, le saccharose, le tréhalose, les dérivés éthylés et méthylés de ces sucres et les composés structurellement analogues, tels que les polyols.

10. Procédé selon l'une quelconque des revendications 1 à 9, **caractérisé en ce que** le composé donneur d'acyle(s) est choisi dans le groupe formé par les acides gras, saturés ou insaturés, à nombre pair ou impair d'atomes de carbones, linéaires ou branchés et dont le nombre d'atomes de carbones est supérieur à 4, les esters d'acides gras, les monoglycérides, diglycérides, triglycérides et les huiles.

11. Procédé selon l'une quelconque des revendications 1 à 10, **caractérisé en ce que** le solvant organique consiste en un solvant choisi dans le groupe formé par le méthanol, l'éthanol, le propanol, le butanol, l'acétone, le propanone, le butanone, le pentanone 2, le 1,2 éthanediol, le 2,3 butanediol, le dioxane, l'acétonitrile, le méthyl 2 butanol 2, le tertiobutanol, le méthyl-2-propanol et l'hydroxy-4-méthyl-2-pentanone, ou un mélange de deux ou de plusieurs de ces solvants.

12. Procédé selon l'une quelconque des revendications I à 11, **caractérisé en ce que** le catalyseur enzymatique consiste en une protéase ou une lipase, préférentiellement immobilisée sur un support.

## Patentansprüche

1. Verfahren zur enzymatischen Synthese von Zuckerestern, **dadurch gekennzeichnet, daß** man in ein geeignetes Reaktionsgefäß zur Herstellung eines Reaktionsmediums bestimmte Mengen eines organischen Lösungsmittels, eines Zuckers oder Zuckerderivats, eines Acyldonators und eines Enzyms als Katalysator einbringt, wobei die Menge mindestens eines Bestandteils dieses Reaktionsmediums im Unterschuß vorliegt, während des Reaktionsverlaufs auf kontrollierte Weise zusätzliche Mengen des oder der im Unterschuß vorliegenden Bestandteils/e zugibt und schließlich die erhaltenen Zuckerester mindestens durch Abtrennen der Enzymteilchen und des Lösungsmittels reinigt.

2. Verfahren nach Anspruch 1, **dadurch gekennzeichnet, daß** man kontinuierlich oder periodisch zusätzliche bestimmte Mengen an Zucker oder Zuckerderivat in Form eines Festkörpers oder einer flüssigen Lösung, an Lösungsmittel, an Enzymkatalysator in löslicher oder immobilisierter Form und/oder an Acyldonor, und zwar allein oder in dem Lösungsmittel solubilisiert, zu dem Reaktionsmedium gibt.

3. Verfahren nach Anspruch 1 oder 2, **dadurch gekennzeichnet, daß** man . mindestens einen der Reaktionsmediumbestandteile periodisch oder kontinuierlich abzieht, wobei man den abgezogenen Bestandteil bzw. die abgezogenen Bestandteile nach Fraktionierung wieder in den Reaktor zurückführt.

4. Verfahren nach Anspruch 3, **dadurch gekennzeichnet, daß** man das gesamte Reaktionsmedium periodisch oder kontinuierlich abzieht, wobei ein Bestandteil bzw. mehrere Bestandteile dieses abgezogenen Mediums nach Fraktionierung wieder in das Reaktionsgefäß eingespritzt wird bzw. werden.

5. Verfahren nach einem der Ansprüche 1 bis 4, **dadurch gekennzeichnet, daß** man das Molverhältnis zwischen Zucker(n) oder Zuckerderivat(en) und Acyldonator(en) in dem Reaktionsmedium so kontrolliert, daß dieses Molverhältnis während des gesamten Verfahrensablaufs je nach den betreffenden Bestandteilskombinationen aus Zucker(n) oder Zuckerderivat(en) und Acyldonator(en) 0,01 bis 10,00, vorzugsweise 0,02 bis 1,00 beträgt.

6. Verfahren nach einem der Ansprüche 1 bis 5, **dadurch gekennzeichnet, daß** die Temperatur während der Reaktion auf 20°C bis 100°C und der Partialdruck oberhalb des Reaktionsmediums auf 10 mbar bis 1000 mbar eingestellt wird und das Reaktionsmedium leicht bewegt wird.

7. Verfahren nach einem der Ansprüche 1 bis 6, **dadurch gekennzeichnet, daß** man die verbleibenden Zucker oder Fettkörper durch Extraktion mit organischen Lösungsmitteln oder mit überkritischen Flüssigkeiten entfernt.

8. Verfahren nach einem der Ansprüche 1 bis 7, **dadurch gekennzeichnet, daß** man die erhaltenen Monoester und/oder Diester durch Fällungen oder durch chromatographische Trennungen fraktioniert.

9. Verfahren nach einem der Ansprüche 1 bis 8, **dadurch gekennzeichnet, daß** der Zucker oder das Zuckerderivat aus der Gruppe Fructose, Glucose, Saccharose, Trehalose, Ethylderivate und Methylderivate dieser Zucker und strukturmäßig analoge Verbindungen, wie die Polyole ausgewählt wird.

10. Verfahren nach einem der Ansprüche 1 bis 9, **dadurch gekennzeichnet, daß** der Acyldonator aus der Gruppe der geradkettigen oder verzweigten gesättigten oder ungesättigten Fettsäuren mit gerader oder ungerader Anzahl Kohlenstoffatomen, die über 4 Kohlenstoffatome aufweisen, der Fettsäureester, der Monoglyceride, Diglyceride und Triglyceride sowie der Öle ausgewählt wird.

11. Verfahren nach einem der Ansprüche 1 bis 10, **dadurch gekennzeichnet, daß** das organische Lösungsmittel aus einem der Gruppe Methanol, Ethanol, Propanol, Butanol, Aceton, Propanon, Butanon, 2-Pentanon, 1,2-Ethandiol, 2,3-Butandiol, Dioxan, Acetonitril, 2-Methyl-2-butanol, tert.-Butanol, 2-Methylpropanol, 4-Hydroxy-2-methylpentanon oder einer Mischung von zwei oder mehreren dieser Lösungsmittel besteht.

12. Verfahren nach einem der Ansprüche 1 bis 11, **dadurch gekennzeichnet, daß** der Enzymkatalysator aus einer Protease oder einer Lipase, vorzugsweise aus einer auf einem Träaer immobilisierten Protease oder Lipase, besteht.

## Claims

1. Method for the enzymatic synthesis of sucrose esters, **characterised in that** it consists in introducing given quantities of an organic solvent, a sugar or sugar derivative, an acyl donor compound, and an enzymatic catalyst into an appropriate reactor in such a way as to form a reaction medium, if there is a deficient quantity of at least one of the constituents of said reaction medium, in adding in a controlled manner over the course of the reaction additional quantities of the deficient constituent(s), and, finally, in purifying the sucrose esters produced at least by separating the enzymatic particles and the solvent.

2. Method according to claim 1, **characterised in that** it consists in continuously or intermittently adding to the reaction medium additional given quantities of sugar or sugar derivative, in solid form or in liquid solution form, solvent, enzymatic catalyst, in soluble or immobilised form, and/or an acyl donor compound, alone or solubilised in the solvent.

3. Method according to any one of claims 1 and 2, **characterised in that** it consists in intermittently or continuously removing at least one of the constituents of the reaction medium, said removed constituent(s) being reintroduced into the reactor after fractionation.

4. Method according to claim 3, **characterised in that** it consists in intermittently or continuously removing the whole medium, one or more constituents of said removed medium being re-injected into the reactor after fractionation.

5. Method according to any one of claims 1 to 4, **characterised in that** it consists in monitoring the molar ratio [sugar(s) or sugar derivative(s)/acyl donor(s)] in the reaction medium in such a way that the molar ratio is between 0.01 and 10.00, preferably between 0.02 and 1.00, throughout the course of said method, as a function of the components of the relevant sugar(s) or sugar derivative(s)/acyl donor pair.

6. Method according to any one of claims 1 to 5, **characterised in that** during the reaction the temperature is set between 20° C and 100° C, the partial pressure above the reaction medium is set between 10 mbar and 1000 mbar, and said reaction medium is subjected to mild agitation.

7. Method according to any one of claims 1 to 6, **characterised in that** it consists in eliminating residual sugars or fats by extraction with organic solvents or supercritical fluids.

8. Method according to any one of claims 1 to 7, **characterised in that** it consists in fractionating monoesters and/or diesters produced by precipitation or chromatographic separation.

9. Method according to one any of claims 1 to 8, **characterised in that** the sugar or sugar derivative is selected from the group consisting of fructose, glucose, saccharose, trehalose, ethyl and methyl derivatives of these sugars, and structurally similar compounds, such as polyols.

10. Method according to any one of claims 1 to 9, **characterised in that** the acyl donor compound is selected from the group consisting of fatty acids which are saturated or unsaturated with an even or odd number of carbon atoms, linear or branched, with more than 4 carbon atoms, fatty acids esters, monoglycerides, diglycerides, triglycerides and oils.

11. Method according to any one of claims I to 10, **characterised in that** the organic solvent consists of a solvent selected from the group formed by methanol, ethanol, propanol, butanol, acetone, propanone, butanone, pentan-2-one, 1,2-ethanediol, 2,3-butanediol, dioxan, acetonitrile, 2-methyl-butan-2-ol, tertiobutanol, 2-methylpropanol and 4-hydroxy-2-methylpentanone, or a mixture of two or more of these solvents.

12. Method according to any one of claims 1 to 11, **characterised in that** the enzymatic catalyst consists of a protease or lipase, preferably immobilised on a carrier.
